(19) Europäisches Patentamt
European Patent Office
Office européen des brevets

(11) Veröffentlichungsnummer: **0 400 340 A1**

# (12) EUROPÄISCHE PATENTANMELDUNG

(21) Anmeldenummer: 90108202.4

(22) Anmeldetag: 30.04.90

(51) Int. Cl.5: C07F 9/09, A01N 57/02, C07F 9/24, C07F 9/40, C07F 9/165, C07C 255/64

(30) Priorität: 11.05.89 DE 3915401

(43) Veröffentlichungstag der Anmeldung: 05.12.90 Patentblatt 90/49

(84) Benannte Vertragsstaaten: AT BE CH DE DK ES FR GB IT LI NL

(71) Anmelder: BASF Aktiengesellschaft
Carl-Bosch-Strasse 38
D-6700 Ludwigshafen(DE)

(72) Erfinder: Kardorff, Uwe, Dr.
D 3,4
D-6800 Mannheim 1(DE)
Erfinder: Kuekenhoehner, Thomas, Dr.
Seidelstrasse 2
D-6710 Frankenthal(DE)
Erfinder: Theobald, Hans, Dr.
Queichstrasse 6
D-6703 Limburgerhof(DE)
Erfinder: Kuenast, Christoph, Dr.
Salierstrasse 2
D-6701 Otterstadt(DE)
Erfinder: Hofmeister, Peter, Dr.
Bernard-Humblot-Strasse 12
D-6730 Neustadt(DE)

## (54) Oximinophosphor(phosphon)säurederivate, Verfahren zu ihrer Herstellung und ihre Verwendung zur Bekämpfung von Schädlingen.

(57) Oximinophosphor(phosphon)säurederivate der allgemeinen Formel I

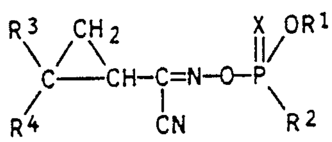

(I)

in der
$R^1$ Alkyl,
$R^2$ Alkyl, Alkoxy, Alkylthio, Phenyl, Amino, Alkyl- oder Dialkylamino,
$R^3$, $R^4$ Wasserstoff, Methyl oder Chlor,
X Sauerstoff oder Schwefel
bedeuten, Verfahren zu ihrer Herstellung sowie ihre Verwendung zur Bekämpfung von Schädlingen und neue α-Oximinonitrile als Zwischenprodukte.

EP 0 400 340 A1

# Oximinophosphor(phosphon)säurederivate, Verfahren zu ihrer Herstellung und ihre Verwendung zur Bekämpfung von Schädlingen

Die vorliegende Erfindung betrifft Oximinophosphor(phosphon)säurederivate der allgemeinen Formel I

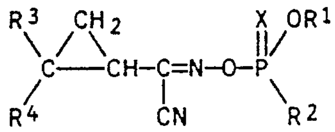

(I)

in der
$R^1$ eine unverzweigte oder verzweigte Alkylgruppe mit 1 bis 4 Kohlenstoffatomen,
$R^2$ eine unverzweigte oder verzweigte Alkylgruppe 1 bis 4 Kohlenstoffatomen, eine unverzweigte oder verzweigte Alkoxygruppe oder Alkylthiogruppe mit 1 bis 4 Kohlenstoffatomen, Phenyl, die Aminogruppe oder einen unverzweigten oder verzweigten Alkylamino- oder Dialkylaminorest mit jeweils 1 bis 4 Kohlenstoffatomen je Alkylgruppe,
$R^3$, $R^4$ Wasserstoff, Methyl oder Chlor und
X Sauerstoff oder Schwefel
bedeuten.

Ferner betrifft die Erfindung ein Verfahren zur Herstellung der Verbindungen I, Schädlingsbekämpfungsmittel, die diese Verbindungen als Wirkstoffe enthalten sowie ein Verfahren zur Bekämpfung von Schädlingen mit diesen Wirkstoffen und Vorprodukte zur Herstellung der Verbindungen I.

Oximinophosphor(phosphon)säurederivate sind aus DE-AS-1 052 981, DE-AS-1 238 902, DE-OS-2 952 738, DE-OS-3 135 182, EP-A-115 318 und EP-A-150 822 bekannt. Thionophosphorsäureoximderivate mit einer $C_5$- oder $C_6$-Cycloalkylgruppe und einer Cyanogruppe an der Oximdoppelbindung sind in DE-OS 23 04 848 zur Bekämpfung von Insekten und Milben offenbart. Da die Wirkung der aus dem o.g. Stand der Technik bekannten Verbindungen unter bestimmten Bedingungen, z.B. niedrigen Aufwandmengen oder Wirkung an bestimmten Problemschädlingen nicht immer befriedigend ist, bestand die Aufgabe, Oximinophosphor(phosphon)säurederivate mit verbesserter Wirkung bereitzustellen.

Demgemäß wurden die eingangs definierten Oximinophosphor(phosphon)säurederivate I sowie Vorprodukte zur Herstellung der Verbindungen I gefunden. Schädlingsbekämpfungsmittel, enthaltend den Wirkstoff I weisen eine starke insektizide, nematizide und akarizide Wirkung auf und sind bekannten Wirkstoffen ähnlicher Struktur bzw. gleicher Wirkungsrichtung überlegen.

Unverzweigte oder verzweigte Alkylreste für $R^1$ sind beispielsweise Methyl, Ethyl, Propyl, Isopropyl, Butyl, Isobutyl, unverzweigte oder verzweigte Alkyl-, Alkoxy- oder Alkylthioreste für $R^2$ sind beispielsweise Methyl, Ethyl, n-Propyl, Isopropyl, Methoxy, Ethoxy, Propoxy- und Butoxyreste, Methylthio, Ethylthio, n-Propylthio, Isopropylthio, n-Butylthio, sec.-Butylthio, Isobutylthio. Als Alkylamino- bzw. Dialkylaminoreste kommen für $R^2$ beispielsweise Methylamino, Dimethylamino, Ethylamino, Diethylamino, Methylethylamino, n-Butylamino, Di-n-butylamino in Betracht.

Bevorzugte Substituenten für $R^1$ sind Methyl und Ethyl; bevorzugte Substituenten für $R^2$ sind Methoxy, Ethoxy, Methyl, Ethyl, Phenyl, Amino, Methylamino, Dimethylamino und Isopropylamino.

Die Oximinophosphor(phosphon)säurederivate der Formel I können durch Umsetzung entsprechender α-Oximinonitrile II mit entsprechenden (Thiono)(Thiol)Phosphor(phosphon)säureester(amid)halogeniden III in Gegenwart eines Säureakzeptors erhalten werden:

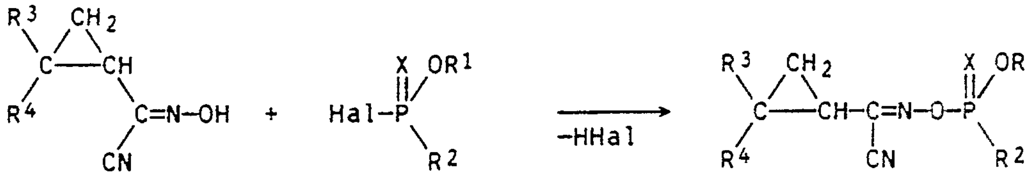


(II) (III) (I)

Aus wirtschaftlichen Gründen wird für Halogen Chlor bevorzugt (Hal = Cl).

Als Säureakzeptor verwendet man für die Phosphorylierung übliche Basen, insbesondere aliphatische,

aromatische oder heterocyclische Amine wie z.B. Dimethylamin, Triethylamin, Diisopropylamin, Piperidin, Dimethylanilin, Dimethylbenzylamin, Pyridin und 4-Dimethylaminopyridin, Alkali- oder Erdalkalimetallcarbonate wie z.B. Natriumcarbonat, Kaliumcarbonat oder Calciumcarbonat, Alkali- oder Erdalkalimetallalkoholate wie z.B. Natriummethylat, Natriumethylat, Calciumethanolat, Kalium-tert.-butylat, Alkali-oder Erdalkalimetallhydride wie z.B. Natriumhydrid, Kaliumhydrid, Calciumhydrid.

Anstelle des Zusatzes eines Säureakzeptors kann man auch vor der Umsetzung der α-Oximinonitrile II die Alkali- oder Erdalkalimetallsalze wie Natrium-, Kalium- oder Calciumsalze oder die Ammoniumsalze herstellen und diese mit Phoshorsäurehalogeniden III umsetzen. Als Lösungs- oder Verdünnungsmittel eignen sich aliphatische, aromatische, gegebenenfalls chlorierte Kohlenwasserstoffe wie z.B. Petrolether, n-Pentan, n-Hexan, Hexan-Isomerengemische, Benzol, Toluol, Xylole, Benzin, Dichlormethan, Chloroform, Tetrachlorkohlenstoff, 1,2-Dichlorethan, Chlorbenzol; Ether wie z.B. Diethylether, Di-n-butylether, Methyl-tert. butylether, Tetrahydrofuran, Dioxan; Ketone und Ester wie z.B. Aceton, Methylethylketon, Methylisopropylketon, Essigsäureethylester; Nitrile wie Acetonitril und Propionitril, ; aprotische dipolare Lösungsmittel wie Dimethylformamid, Dimethylsulfoxid, Pyridin. Auch Gemische dieser Stoffe können als Lösungs-und Verdünnungsmittel verwendet werden. Üblicherweise setzt man die Ausgangsstoffe im stöchiometrischen Verhältnis ein. Ein Überschuß einer Komponente kann in Einzelfällen durchaus vorteilhaft sein.

Die Umsetzung verläuft im allgemeinen in einem Temperaturbereich von 10° C bis 120° C, vorzugsweise bei Raumtemperatur (20° C) bis 70° C. Im allgemeinen finden die Reaktionen bei Normaldruck statt.

Aus dem Reaktionsgemisch wird der erfindungsgemäße Wirkstoff in üblicher Weise gewonnen, zum Beispiel durch Versetzen mit Wasser, Trennen der Phasen und Destillation und/oder Säulenchromatographie. Die zur Herstellung von Verbindungen der Formel I als Ausgangsmaterialien verwendeten α-Oximinonitrile II sind neu. Sie lassen sich in an sich bekannter Weise (vgl. DE-AS-15 67 142) durch Chlorieren der zugehörigen Oxime IV und anschließende Umsetzung mit Natrium- oder Kaliumcyanid gemäß folgender Reaktionsgleichung herstellen:

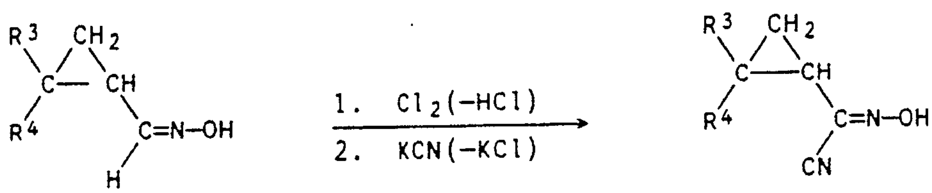


(IV) (II)

Die Oxime der Formel IV erhält man durch Umsetzung des entsprechenden Cyclopropancarboxaldehyds der Formel V mit Hydroxylaminhydrochlorid nach folgender Reaktionsgleichung:

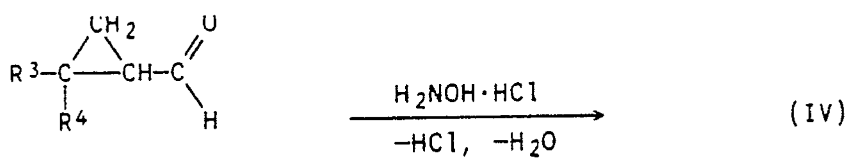


(V)

Cyclopropylcarboxaldehyd ($R^3,R^4 = H$) ist im Chemikalienhandel erhältlich, 1,1-Dichlorcyclopropancarboxaldehyd ($R^3,R^4 = Cl$) ist literaturbekannt (vgl. A. Kh. Khusid, Zh. Org. Khim. 1986, 22 (6), 1195-1200).

Die zur Synthese der Verbindungen der Formel I außerdem benötigten (Thiono)(Thiol)Phosphor(phosphon)säureester(amid)halogenide III sind aus Houben-Weyl, Methoden der organischen Chemie, Band 12/2, S. 274 ff, Georg-Thieme-Verlag, Stuttgart 1964, bekannt und lassen sich auf den dort beschriebenen Synthesewegen herstellen.

Die neuen Verbindungen der Formel I fallen teilweise in Form farbloser oder schwach bräunlich gefärbter Öle an, die sich durch längeres Erwärmen unter verminderten Druck auf mäßig erhöhte Temperatur ("Andestillieren") von den letzten flüchtigen Anteilen befreien und auf diese Weise reinigen lassen. Sind die Verbindungen der Formel I kristallin, so kann ihre Reinigung durch Umkristallisation erfolgen.

Da die Verbindungen der Formel I meist als Gemische der syn- und anti-Formen auftreten, eignen sich deren Schmelz- bzw. Siedebereiche wenig zur Identifizierung. Von den jeweils hergestellten Stoffen sind daher die Absorptionsmaxima der IR-Spektren im sogenannten "fingerprint"-Bereich kleiner 1500 $cm^{-1}$

angegeben.

Beispiel 1

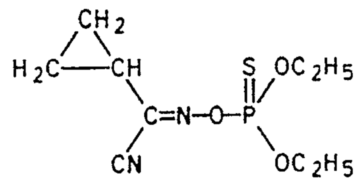

3,3 g (0,03 mol) 2-Cyclopropyl-2-hydroximinoacetonitril werden in 35 $cm^3$ Acetonitril gelöst, mit 4,14 g Kaliumcarbonat versetzt und 6,56 g Thiophosphorsäure-O,O-diethylesterchlorid zugetropft. Die Mischung wird 14 h bei Raumtemperatur gerührt, dann von unlöslichen Bestandteilen über eine Fritte abgesaugt und das Filtrat im Vakuum eingeengt. Der Rückstand wird in Methyl-tert.-butylether aufgenommen, mit 5 %iger wäßriger Natronlauge und mit Wasser gewaschen, über Natriumsulfat getrocknet und vom Lösungsmittel befreit. Nach dem Andestillieren bei 50°C und 0,1 mbar verbleiben 7,6 g (O,O-Diethylthiophosphoryl)-oximino-2-cyclopropylacetonitril als leicht viskoses Öl.
Ausbeute: 97 % der rechnerisch möglichen
Elementaranalyse: $C_9H_{15}N_2O_3$ PS

| | | | |
|---|---|---|---|
| ber.: | C: 41,22; | H: 5,77; | N: 10,68; |
| gef.: | C: 41,4 ; | H 6,0 ; | N: 11,3 ; |

300 MHz-$^1$H-NMR-Spektrum in $CDCl_3$ ($\delta$ -Werte in ppm gegen Tetramethylsilan als internen Standard); 1,01-1,20 (m,4H), 1,39 (t,6H); 2,03-2,13 (m,1H); 4,20-4,36 (m,4H)
Infrarotabsorptionen ($cm^{-1}$): 1164, 1045, 1022, 975, 933, 904, 852.

Beispiel 2

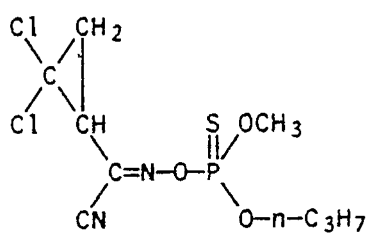

2,69 g (15 mmol) 2(1,1-Dichlor)cyclopropyl-2-hydroximinoacetonitril werden in 25 $cm^3$ Acetonitril gelöst, 2,07 g Kaliumcarbonat hinzugefügt und 2,83 g Thiophosphorsäure-O-methylester-0-n-propylesterchlorid zugetropft. Bei Raumtemperatur wird ca. 50 h gerührt. Nach dem Abfiltrieren wird das Acetonitril abdestilliert, der Rückstand in Methyl-tert. butylether aufgenommen, mit 5 %iger wäßriger Natronlauge und mit Wasser gewaschen, über Natriumsulfat getrocknet und im Vakuum eingeengt. Säulenchromatographie mit 1,2-Dichlorethan/Cyclohexan als Laufmittelgemisch ergibt 4,4 g (O-Methyl-O-n-propylthiophosphoryl)-oximino-2-(1,1-dichlor)-cyclopropylacetonitril als leicht viskoses Öl.
Ausbeute: 89 % der rechnerisch möglichen.
Elementaranalyse: $C_9H_{13}Cl_2N_2O_3PS$

| | | | |
|---|---|---|---|
| ber.: | C: 32,64; | H: 3,96; | N: 8,46 |
| gef.: | C: 32,8 ; | H: 4,0 ; | N: 8,8 |

250 MHz-$^1$H-NMR-Spektrum in $CDCl_3$($\delta$-Werte in ppm gegen Tetramethylsilan): 0,95 (t,3H); 1,70-1,85 (m,2H); 2,09-2,28 (m,2H); 2,83 (dd,1H), 3,90 (d,3H); 4,10-4,27 (m,2H).
Infrarotabsorptionen ($cm^{-1}$): 1109, 1044, 1017, 976, 917, 865, 828, 774.

Beispiel 3

Darstellung von 2-Cyclopropyl-2-hydroximinoacetonitril

21,6 g (0,254 mol) Cyclopropylcarbaldoxim und 35 g Kaliumcarbonat werden in 0,9 l trockenem Methylenchlorid bei -20 °C vorgelegt und während ca. 50 min mit 19,84 g Chlorgas versetzt. Nach dem Erwärmen auf Raumtemperatur wird das Methylenchlorid abgezogen und der Rückstand in 0,5 l Diethylether aufgenommen.

Diese etherische Lösung wird bei 10 bis 15 °C zu einer Suspension aus 18,2 g Kaliumcyanid in 190 ml Methanol zugetropft und das Reaktionsgemisch 3 h bei Raumtemperatur nachgerührt. Der Niederschlag wird abfiltriert und das Filtrat bis zur Trockene eingeengt. Der Rückstand wird in Methyl-tert.-butylether aufgenommen, mit Wasser gewaschen, über Natriumsulfat getrocknet und das Lösungsmittel abdestilliert. Das so erhaltene Rohprodukt wird durch Säulenchromatographie (Kieselgel, Cyclohexan/Methyl-tert.-butylether als Laufmittelgemisch) gereinigt.

Ausbeute: 22,1 g, entsprechend 79 % der rechnerisch möglichen Menge eines hellgelben Öls, Kp.: 134 °C/0,4 mbar.
Das Produkt liegt als syn/anti-Gemisch vor.

250 MHz-$^1$H-NMR-Spektrum in $d^6$-DMSO ($\delta$-Werte in ppm gegen Tetramethylsilan als internen Standard);
0,72-1,16 (m, 4H), 1,85-2,0 und 2,3-2,4 (m, 1H), 12,8 und 13,0 (s, 1H).
IR-Absorptionen ($cm^{-1}$): 1583, 1426, 1337, 1271, 1062, 1030, 993.

Die in der nachstehenden Tabelle aufgeführten Verbindungen wurden auf den in Beispiel 1 bzw. Beispiel 2 beschriebenen Wegen erhalten, soweit mindestens eine physikalische Angabe zu ihrer Identifizierung gemacht ist; andere Verbindungen, die der Formel I entsprechen, können auf die gleiche Weise unter entsprechender Abwandlung der Vorschriften, gegebenenfalls nach einem Vorversuch, um die besten Reaktionsbedingungen zu finden, erhalten werden.

Tabelle

Oximinophosphor(phosphon)säurederivate der Formel I

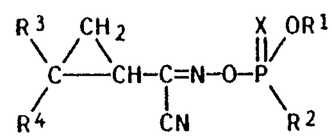

(I)

| Bsp. Nr. | $R^1$ | $R^2$ | $R^3$ | $R^4$ | X | IR-Absorptionen (fingerprint-Bereich) $cm^{-1}$ |
|---|---|---|---|---|---|---|
| 3 | $CH_3$ | $OCH_3$ | H | H | S | 1457, 1181, 1036, 933, 905, 860, 828, 735, 645. |
| 4 | $CH_3$ | $OCH_3$ | H | H | O | |
| 5 | $CH_3$ | $C_2H_5$ | H | H | S | 1048, 1026, 924, 884, 843, 819. |
| 6 | $CH_3$ | $OC_2H_5$ | H | H | S | 1164, 1026, 978, 934, 904, 857, 830, 796, 734, 642. |
| 7 | $CH_3$ | $On-C_3H_7$ | H | H | S | 1461, 1046, 1016, 934, 905, 860, 826, 754, 734. |
| 8 | $CH_3$ | $Oi-C_3H_7$ | H | H | S | 1180, 1047, 1012, 933, 902, 855, 829, 778, 732. |
| 9 | $C_2H_5$ | $CH_3$ | H | H | S | |
| 10 | $C_2H_5$ | $CH_3$ | H | H | O | |
| 11 | $C_2H_5$ | $C_2H_5$ | H | H | S | 1046, 1023, 963, 933, 883, 840, 798, 769, 757. |
| 12 | $C_2H_5$ | $OC_2H_5$ | H | H | O | 1368, 1290, 1167, 1098, 1030, 988, 934, 854, 762. |

Tabelle (Fortsetzung)

| Bsp. Nr. | $R^1$ | $R^2$ | $R^3$ | $R^4$ | X | IR-Absorptionen (fingerprint-Bereich) $cm^{-1}$ |
|---|---|---|---|---|---|---|
| 13 | $C_2H_5$ | $On-C_3H_7$ | H | H | S | 1015,980,933,904,857,813,797,752,733. |
| 14 | $C_2H_5$ | $Sn-C_3H_7$ | H | H | S | 1023,964,924,895,841,789 |
| 15 | $C_2H_5$ | $Sn-C_3H_7$ | H | H | O | 1269,1026,968,929,903,843,725,624,602. |
| 16 | $C_2H_5$ | $Oi-C_3H_7$ | H | H | S | 1388,1045,1009,933,901,852,819 |
| 17 | $C_2H_5$ | $NH_2$ | H | H | O | |
| 18 | $C_2H_5$ | $NH-i-C_3H_7$ | H | H | S | 1412,1134,1042,934,901,846,825 |
| 19 | $C_2H_5$ | $NH-iC_3H_7$ | H | H | O | |
| 20 | $C_2H_5$ | $On-C_4H_9$ | H | H | S | 1045,1022,981,933,903,852,819,735 |
| 21 | $C_2H_5$ | $Osec-C_4H_9$ | H | H | S | 1045,1011,960,933,903,861,853,821,796 |
| 22 | $C_2H_5$ | $Oi-C_4H_9$ | H | H | S | 1046,1017,977,933,904,872,814,797,733. |
| 23 | $C_2H_5$ | $Ssec-C_4H_9$ | H | H | S | |
| 24 | $C_2H_5$ | $Ssec-C_4H_9$ | H | H | O | |
| 25 | $C_2H_5$ | $C_6H_5$ | H | H | S | |
| 26 | $nC_3H_7$ | $On-C_3H_7$ | H | H | S | |
| 27 | $iC_3H_7$ | $Oi-C_3H_7$ | H | H | S | |
| 28 | $CH_3$ | $OCH_3$ | Cl | Cl | S | 1182,1109,1038,977,919,862,831 |
| 29 | $CH_3$ | $OCH_3$ | Cl | Cl | O | |
| 30 | $CH_3$ | $C_2H_5$ | Cl | Cl | S | 1110,1054,1026,973,915,897,844,815 |
| 31 | $CH_3$ | $OC_2H_5$ | Cl | Cl | S | 1164,1109,1029,977,919,858,831 |

Tabelle (Fortsetzung)

| Bsp. Nr. | $R^1$ | $R^2$ | $R^3$ | $R^4$ | X | IR-Absorptionen (fingerprint-Bereich) $cm^{-1}$ |
|---|---|---|---|---|---|---|
| 32 | $CH_3$ | $Oi-C_3H_7$ | Cl | Cl | S | 1108, 1052, 1011, 974, 918, 898, 857, 829, 775 |
| 33 | $C_2H_5$ | $CH_3$ | Cl | Cl | S | |
| 34 | $C_2H_5$ | $CH_3$ | Cl | Cl | O | |
| 35 | $C_2H_5$ | $C_2H_5$ | Cl | Cl | S | 1109, 1048, 1022, 968, 941, 914, 899, 840, 798 |
| 36 | $C_2H_5$ | $OC_2H_5$ | Cl | Cl | S | 1163, 1108, 1022, 978, 917, 852, 824, 800 |
| 37 | $C_2H_5$ | $OC_2H_5$ | Cl | Cl | O | |
| 38 | $C_2H_5$ | $On-C_3H_7$ | Cl | Cl | S | 1108,1015,977,917,885,858,813 |
| 39 | $C_2H_5$ | $Sn-C_3H_7$ | Cl | Cl | S | 1109,1022,968,912,840 |
| 40 | $C_2H_5$ | $Sn-C_3H_7$ | Cl | Cl | O | |
| 41 | $C_2H_5$ | $Oi-C_3H_7$ | Cl | Cl | S | |
| 42 | $C_2H_5$ | $NH_2$ | Cl | Cl | O | |
| 43 | $C_2H_5$ | $NH-i-C_3H_7$ | Cl | Cl | S | 1134,1040,971,916,845,827 |
| 44 | $C_2H_5$ | $NH-i-C_3H_7$ | Cl | Cl | O | |
| 45 | $C_2H_5$ | $On-C_4H_9$ | Cl | Cl | S | |
| 46 | $C_6H_5$ | $Osec-C_4H_9$ | Cl | Cl | S | |
| 47 | $C_2H_5$ | $Oi-C_4H_9$ | Cl | Cl | S | |
| 48 | $C_2H_5$ | $Ssec-C_4H_9$ | Cl | Cl | S | |
| 49 | $C_2H_5$ | $Ssec-C_4H_9$ | Cl | Cl | O | |
| 50 | $C_2H_5$ | $C_6H_5$ | Cl | Cl | S | |
| 51 | $n-C_3H_7$ | $On-C_3H_7$ | Cl | Cl | S | |
| 52 | $i-C_3H_7$ | $Oi-C_3H_7$ | Cl | Cl | S | |
| 53 | $CH_3$ | $On-C_3H_7$ | Cl | Cl | S | 1109,1044,1017,976,917,865,828,724 |
| 54 | $C_2H_5$ | $Oi-C_3H_7$ | Cl | Cl | S | 1108,1009,977,918,897,870 |

Die Oximinophosphor(phosphon)säurederivate der Formel I sind geeignet, Schädlinge aus der Klasse der Insekten, Spinnentiere und Nematoden wirksam zu bekämpfen. Sie können im Pflanzenschutz sowie auf den Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans. Oestrus ovis, Oscinella frit, Pegomya hysocyami, Phorbia antiqua, Phorbia brassicae, Phorbia coarctata. Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 4 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 13 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol

Ricinusöl besteht.

V. 80 Gew.-Teile der Verbindung Nr. 19 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,2 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden aus DE-A-23 04 848 bekannten Verbindungen A und B verglichen.

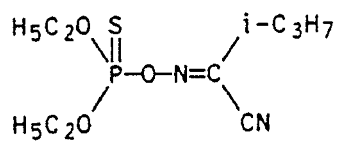

A

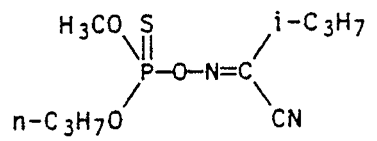

B

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche durchgeführt.

Die Reinheit der Wirkstoffe liegt >95 %. Als Formulierung wurde entweder eine acetonische Lösung des Wirkstoffs oder ein 10 gew.-%iges Emulsionskonzentrat gewählt, das durch Emulgieren des Wirkstoffes in einer Mischung aus 70 Gew.-% Cyclohexanon, 20 Gew.-% Nekanil LN® (≙ Lutensol AP6, Netzmittel mit Emulgier- und Dispergierwirkung auf Basis ethoxylierter Alkylphenole) und 10 Gew.-% Emulphor EL® (≙ Emulan EL®, Emulgator auf Basis ethoxylierter Fettalkohole) erhalten wurde, gewählt. Die in den Beispielen angegebenen Konzentrationen wurden durch Verdünnen des formulierten Wirkstoffes mit Wasser erhalten.

Kontaktwirkung auf Blatta orientalis (Schabe)

Der Boden eines 1-l-Glases wird mit der acetonischen Lösung des Wirkstoffes behandelt. Nach Verdunsten des Lösungsmittels setzt man in jedes Glas 5 adulte Schaben.

Die Mortalitätsrate wird nach 48 Stunden bestimmt.

Mit den Verbindungen der Beispiele 1, 3, 6-8, 11-13, 16, 20-22, 28, 30-32, 35, 36, 38, 39, 43, 53 und 54 wird eine bessere Wirkung erzielt als mit den Vergleichsmitteln A und B.

Kontaktwirkung auf Sitophilus granaria (Kornkäfer)

Aufgerauhte Glasplatten von 8 × 8 cm Kantenlänge werden mit der acetonischen Wirkstofflösung behandelt.

Nach Verdunsten des Lösungsmittels belegt man die Platten mit 100 Kornkäfern und deckt mit einem Uhrglas (Ø6 cm) ab. Nach 4 Stunden werden die Käfer in unbehandelte Gefäße überführt. Die Mortalitätsrate wird nach 24 Stunden ermittelt. Dabei wird festgestellt, wieviele Käfer in der Lage waren, nach diesem Zeitpunkt innerhalb von 60 Minuten ein unbehandeltes Pappschälchen (Ø40 mm, Höhe 10 mm) zu verlassen.

Mit den Verbindungen der Beispiele 1-3, 5-8, 11-13, 15, 16, 18, 20, 21, 30-32, 35, 36, 38, 53 und 54 wird eine bessere Wirkung erzielt als mit den Vergleichsmitteln A und B.

Dauerkontakt auf Musca domestica (Stubenfliegen)

Beide Teile einer Petrischale von 10 cm Durchmesser werden mit insgesamt 2 ml der acetonischen Wirkstofflösung ausgekleidet. Nach Verdunsten des Lösungsmittels (ca. 30 Minuten) bringt man je 10 Fliegen in die Schalen. Die Mortalität wird nach 4 Stunden festgestellt.

Hierbei erzielen die Wirkstoffe der Beispiele 1, 2, 6-8, 11-13, 16, 20-22, 28, 30, 31, 32, 35, 36, 38, 39, 43, 53 und 54 eine bessere Wirkung als die Vergleichsmittel A und B.

Plutella maculipennis (Kohlschauben-Raupen), Fraßwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt. Nach 48 Stunden beurteilt man die Wirkung.

Hierbei wird mit den Wirkstoffen 1-3, 5-8, 11-14, 16, 20-22, 28, 30-32, 35, 36, 38, 39, 53 und 54 eine bessere Wirkung als mit Vergleichsmittel A erzielt.

Kontaktwirkung auf Prodenia litura (Ägyptischer Baumwollwurm)

Der Boden von Glaspetrischalen (Ø10 cm) wird mit der acetonischen Wirkstofflösung behandelt. Nach dem Verdunsten des Lösungsmittels besetzt man die Schalen mit 5 Raupen des 3. Stadiums und verschließt sie. Nach 4 Stunden wird die Mortalität in % festgestellt.

Hierbei erzielen die Verbindungen der Beispiele 1, 3, 6-8, 11, 14, 15, 18, 20, 28, 30-32, 35, 36, 38, 39, 43 und 54 eine bessere Wirkung als die Vergleichsmittel A und B.

## Ansprüche

1. Oximinophosphor(phosphon)säurederivate der allgemeinen Formel I

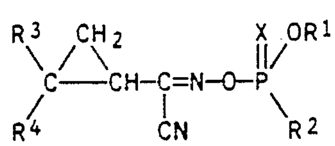

(I)

in der

$R^1$ eine unverzweigte oder verzweigte Alkylgruppe mit 1 bis 4 Kohlenstoffatomen,

$R^2$ eine unverzweigte oder verzweigte Alkylgruppe 1 bis 4 Kohlenstoffatomen, eine unverzweigte oder verzweigte Alkoxygruppe oder Alkylthiogruppe mit 1 bis 4 Kohlenstoffatomen, Phenyl, die Aminogruppe oder einen unverzweigten oder verzweigten Alkylamino- oder Dialkylaminorest mit jeweils 1 bis 4 Kohlenstoffatomen je Alkylgruppe,

$R^3$, $R^4$ Wasserstoff, Methyl oder Chlor und

X Sauerstoff oder Schwefel

bedeuten.

2. Verfahren zur Herstellung von Oximinophosphor(phosphon)säurederivaten der Formel I gemäß Anspruch 1, dadurch gekennzeichnet, daß man $\alpha$-Oximinonitrile der Formel II

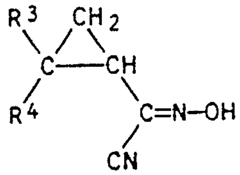

(II)

in der $R^3$ und $R^4$ die in Anspruch 1 genannte Bedeutung haben, ggf. in Gegenwart eines Säureakzeptors oder Alkalimetall-, Erdalkalimetall-oder ggf. substituierte Ammoniumsalze dieser $\alpha$-Oximinonitrile mit (Thiono)(Thiol)phosphor(phosphon)säureester(amid)halogeniden der Formel III

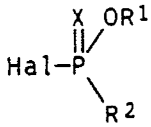

(III)

umsetzt.

3. Schädlingsbekämpfungsmittel, enthaltend einen festen oder flüssigen Trägerstoff und mindestens ein Oximinophosphor(phosphon)säurederivat der Formel I gemäß Anspruch 1.

4. Verfahren zur Bekämpfung von Schädlingen, dadurch gekennzeichnet, daß man Oximinophosphor(phosphon)säurederivate der Formel I gemäß Anspruch 1 auf Schädlinge bzw. deren Lebensraum einwirken läßt.

5. Verwendung von Oximinophosphor(phosphon)säurederivaten der Formel I gemäß Anspruch 1 zur Bekämpfung von Schädlingen.

6. $\alpha$-Oximinonitrile der Formel II

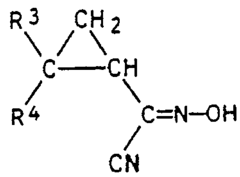

(II),

in der $R^3$ und $R^4$ Wasserstoff, Methyl oder Chlor bedeuten.

Europäisches Patentamt

# EUROPÄISCHER RECHERCHENBERICHT

Nummer der Anmeldung

EP 90 10 8202

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br>Y | EP-A-0 129 889 (NIPPON KAYAKU K.K.)<br>* Ansprüche *<br>--- | 1-6<br>1-5 | C 07 F 9/09<br>A 01 N 57/02<br>C 07 F 9/165<br>C 07 F 9/24<br>C 07 F 9/40<br>C 07 C 255/64 |
| Y | EP-A-0 115 318 (BASF)<br>* Insgesamt *<br>----- | 1-5 | |
| | | | **RECHERCHIERTE SACHGEBIETE (Int. Cl.5)** |
| | | | C 07 F 9/00<br>A 01 N 57/00<br>C 07 C 255/00 |

Der vorliegende Recherchenbericht wurde für alle Patentansprüche erstellt

| Recherchenort | Abschlußdatum der Recherche | Prüfer |
|---|---|---|
| DEN HAAG | 25-09-1990 | OUSSET J-B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument